# EUROPEAN PATENT SPECIFICATION

(11) **EP 2 747 645 B1**
(45) Date of publication and mention of the grant of the patent: **22.02.2017**
(21) Application number: 12808470.4
(22) Date of filing: 09.08.2012
(51) Int. Cl.: A61B 5/103

(54) **SENSORIZED MAT STRUCTURE**
MATTENSTRUKTUR MIT SENSOREN
STRUCTURE DE MATELAS À CAPTEURS

(30) Priority: 23.08.2011 IT PI20110091
(43) Date of publication of application: 02.07.2014
(73) Proprietor: Scuola Superiore di Studi Universitari e di Perfezionamento Sant'Anna, 56127 Pisa (IT)
(72) Inventor: DE ROSSI, Stefano Marco Maria, 30035 Mirano (VE) (IT); LENZI, Tommaso, 56025 Pontedera (PI) (IT); VITIELLO, Nicola, 56025 Pontedera (PI) (IT); PERSICHETTI, Alessandro, 56031 Bientina (PI) (IT); GIOVACCHINI, Francesco, 56121 Pisa (IT); CARROZZA, Maria Chiara, 56100 Pisa (IT)
(74) Representative: Celestino, Marco
(86) International application number: PCT/IB2012/054068
(87) International publication number: WO 2013/027145

(56) References cited:
- US-A1- 2009 137 933
- US-A1- 2010 076 347
- DE ROSSI S M M ET AL: "Development of an in-shoe pressure-sensitive device for gait analysis", 33RD ANNUAL INTERNATIONAL CONFERENCE OF THE IEEE EMBS, BOSTON, MASSACHUSETTS USA, 2011, 30 August 2011 (2011-08-30), pages 5637-5640, XP032026366, DOI: 10.1109/IEMBS.2011.6091364 ISBN: 978-1-4244-4121-1
- STEFANO MARCO MARIA DE ROSSI ET AL: "Sensing Pressure Distribution on a Lower-Limb Exoskeleton Physical Human-Machine Interface", SENSORS, vol. 11, no. 1, 1 January 2010 (2010-01-01), pages 207-227, XP055020897, ISSN: 1424-8220, DOI: 10.3390/s110100207
- TAO LIU ET AL: "A Wearable Force Plate Designed Using Pressure Sensitive Electric Conductive Rubber", JOURNAL OF SYSTEM DESIGN AND DYNAMICS, vol. 3, no. 3, 1 January 2009 (2009-01-01) , pages 282-295, XP055020898, ISSN: 1881-3046, DOI: 10.1299/jsdd.3.282
- LIN SHU ET AL: "In-Shoe Plantar Pressure Measurement and Analysis System Based on Fabric Pressure Sensing Array", IEEE TRANSACTIONS ON INFORMATION TECHNOLOGY IN BIOMEDICINE, vol. 14, no. 3, 1 May 2010 (2010-05-01), pages 767-775, XP011345692, ISSN: 1089-7771, DOI: 10.1109/TITB.2009.2038904
- WEI-CHIH WANG ET AL: "Development of a Microfabricated Optical Bend Loss Sensor for Distributive Pressure Measurement", IEEE TRANSACTIONS ON BIOMEDICAL ENGINEERING, vol. 55, no. 2, 1 February 2008 (2008-02-01), pages 614-625, XP011200293, ISSN: 0018-9294, DOI: 10.1109/TBME.2007.912627

## Description

### Field of the invention

The present invention relates to a sensorized mat structure capable of measuring the action of contact with a foot sole of a foot of a user, determining the intensity of the tactile action and application position of the action same.

### Description of the prior art

As well known, for analysing a static standing position a patient or even for evaluating the dynamic of the gait, sensorized mats are used where the plantar pressures in the many zones of the foot, or the variation and the distributions of the plantar pressures during the gait, are monitored. A specific kind of such mats for evaluating the plantar pressures is known and is called footboard or force platform.

The force platform is a mat consisting of a plurality of sensors that measure, during the movement of the foot, a force, a surface and a time. These three factors allow to analyse the set of the movement of the lower limbs and to ascertain the possible postural origin of the symptoms described by the patient.

Alternatively to sensorized mats, so-called sensorized insoles are used, which allow to analyse the pressures of a foot in a shoe, recording data on the basis also of a few walking steps up to several walking hours. The patient is free of moving in any direction and according to the own habits.

In particular, each sensorized insole is put in the shoe and is connected to a data-collection computing unit born by the shoe of the patient. Alternatively, where not possible, the electronics can be tied to the pelvis, to the ankle, to the leg, or it can be connected by a cable to the insole.

In particular, various types of remote sensors are used for the insoles such as capacitive sensors, in which the pressure of the foot changes the capacity (see for example WO2009089406), resistive sensors, in which the pressure of the foot changes the resistance, ink sensors or polymers sensors that exploit the conductive capacity of inks or polymers. The above described types of sensors require frequent adjustment, in order to obtain accurate measurements.

A hydrocell technology is also known, where on the support surface of the foot bearings containing liquid, or air, are arranged, combined with used piezoresistive pressure sensors for determining the fluid pressure.

Further sensors are used comprising optical sensors that measure the variation of emitted light by a source towards a sensitive element.

The mats that make the sensorized insoles can be classified both for kind of sensor used and for number and distribution of sensors and comprise: mats or insoles to discrete distribution and mats or insoles with matrix of sensors.

In the first kind the number of sensors does not cover the whole area of the mat or insole, and it is possible to determine only one information relative to the vertical component of the force of reaction to the ground in the areas of interest, i.e. the areas where the sensors are provided.

The above described kind, owing to a low number of sensors, leaves free space within the same mat or insole to implement all the electronics necessary to their operation, such that they are of comfortable and practical use, but does not allow to obtain a detection of the pressures.

In the second kind, i.e. mats or insoles with matrix of sensors, as described in WO2009089406, a much higher number of sensors is present that cover substantially all the area of contact. This way, it is possible to obtain an overall mapping of the plantar pressure, in particular: the vertical component of the total force of reaction to the ground *(Total ground reaction force*)*,* the centre of pressure (CoP) and the distribution of the plantar pressures on all the foot. It can occur, however, that in such mats or insoles the matrix arrangement of the sensors, and then an arrangement close to each other, involves, for a proximity effect, that neighbouring sensors that are not directly subject to the plantar pressure (cross-talk) are affected. A non accurate measurement follows that is not reliable since it involves a number of sensors different with respect to those actually subject to the plantar pressure.

In addition, in the above described types of mats and insoles with high density of sensors, it is necessary to provide a separated and external electronics for analysing and amplifying the signals coming from the sensors and transmitting them to a remote control unit, as well as a remote battery. Therefore such matrix mats or insoles are exclusively used in specialized centres since they require dedicated spaces and devices that have to be assembled and tuned by expert operators to obtain reliable measurements.

This makes impossible a use for example in the field of household therapeutic devices or customized devices.

The article by Wei-Chih Wang et al., "Development of a Microfabricated Optical Bend Loss Sensor for Distributive Pressure Measurement", IEEE Transactions on Biomedical Engineering, vol. 55, no. 2, 2008, p. 614-625, discloses a flexible high-resolution sensor capable of measuring the distribution of pressure beneath the foot via a microfabricated optical waveguide system, wherein, as the sensor is loaded, the waveguide is bent and light intensity in the waveguide is attenuated.

The article by Stefano Marco Maria De Rossi et al., "Sensing Pressure Distribution on a Lower-Limb Exoskeleton Physical Human-Machine Interface", Sensors, 2011, vol. 11, p. 207-227, discloses a distributed pressure sensor made of an array of sensitive elements based on a mechano-opto-electronic transduction principle, wherein each sensitive element is composed of a light emitter and of a light receiver, and the whole sensor is covered by a soft silicone shell.

The need is felt to provide a sensorized mat or insole that has not problems of adjustment of the sensors and that does not require external control apparatus or supply means and that makes it possible to obtain precise and reliable measurements.

In particular, in case of an insole it is desirable that is can be completely integrated in the shoes and that it allows in the meantime to obtain a full mapping of the plantar area.

### Summary of the invention

It is therefore a feature of the present invention to provide a structure of sensorized mat that has a high density of sensors and then a good spatial resolution and in the meantime does not require external devices.

It is another feature of the present invention to provide a structure of sensorized mat that makes it possible to make very accurate measurements and that does not require adjustment operations.

It is a further feature of the present invention to provide a structure of sensorized mat that can be inserted completely within a shoe in order to monitor daily walking conditions in a patient.

It is still a feature of the present invention to provide a structure of sensorized mat of easy and cheap production.

These and other objects are achieved by a sensorized mat structure comprising:
- a support base;
- a plurality of sensors distributed on said support base adapted to generate respective measurement signals when they are subjected to a load bearing on it;
- a control unit associated with said plurality of sensors configured to analyse and/or transmit said measurement signals;
- a supply unit arranged to supply said plurality of sensors and said control unit;
wherein each sensor comprises a deformable cell with box-like shape, and wherein said plurality of sensors is arranged on said support base so that said cells are adjacent with respect to each other,
each of said cells comprising:
- a support wall that is opposite, in use, to said support base, and arranged to receive said load, and a plurality of deformable side walls located between said support base and said support wall that define a housing;
- at least one light emitter and at least one photosensitive detector in said housing, said photosensitive detector configured to measure a changing light intensity collected from said emitter
   owing to the deformation of each cell when it is subjected to said load,
- wherein said cells are separated from each other by a predetermined separation space such that, when said support wall of each cell is subjected to said load, said side walls change their shape and are compressed towards said support base and, while expanding laterally, occupy a deformation space less than said separation space, so that adjacent cells do not touch each other;
wherein said cells have a truncated pyramid-structure
and define a matrix of cells, in such a way that:
- said truncated pyramid structure is stiff with respect to tangential loads bearing on said side walls, and deformable with respect to normal loads bearing on said support wall;
- said truncated pyramid structure in combination with said sensors allows that said measurement signals have a broad dynamic range that is high enough to be directly transmitted and analysed by said control unit;
wherein each truncated pyramid cell has a stepped portion in such a way that:
- stepped portions of adjacent truncated pyramid cells are substantially adjacent to each other;
- a minimum separation space Smin is defined at said stepped portion, and a maximum separation space Smax is defined at said support wall;
and wherein each of said cells comprises:
- said emitter of a light source;
- said photosensitive detector arranged opposite said emitter;
- a separation element located between said emitter and said photosensitive detector and that is arranged to move from a rest configuration, in which said cell is not subject to said load and all the light emitted by said emitter is collected by
   said photosensitive detector, to a bearing configuration, in which said separation element is lowered proportionally to said load and is located between said emitter and said photosensitive detector partially masking the light directed towards said photosensitive detector, proportionally to said load.

This way, each cell recovers a measurement signal responsive to the load bearing on it without that there are cross-talk phenomena between adjacent cells. In fact, only the cells subject to the load generate a corresponding measurement signal and adjacent cells are not affected, because the deformation of the side walls of each cell is less than the deformation space set between the cells. In other words, the cells not directly subject to the load are not affected by the deformation or "swelling" of the cells subject to the load, and do not provide then a corresponding measurement signal. It is, thus ensured a most accurate measurement of the pressures generated on the foot sole during the walking or during the maintenance of a static standing position.

In addition, the cells with box-like shape are arranged as an array of resilient dampers allowing an overall damping, without then requiring further insole damping layers.

Advantageously, each cell comprises a boundary frame, of resilient material, to define said housing where they said emitter and said photosensitive detector, said boundary frame arranged to contain electronic components, for example acquisition/conditioning components.

The boundary frame surrounds a housing where the emitter and the detector are arranged, providing a free zone available for arranging in a distributed way electronic components. In fact, for the presence of the side walls and of their thickness, the areas in the sensor are surrounded, seeing them in a top plan view, by the boundary frame not interested by the area occupied by the emitter and by the detector. Such frame provides a zone available in which it is possible to arrange electronic components, for example acquisition/conditioning components, in a distributed way.

In particular, each cell comprises an "empty" part, which represents said housing where said light emitter and said photosensitive detector are housed, and a "solid" part filled of resilient material, in particular silicone rubber, connected to said support base. The solid parts form, seeing them in a top plan view, a sort of "matrix of frames". All this area can be used, advantageously, for housing in a distributed way useful electronic components for example for conditioning or transmitting the obtained data.

In an exemplary embodiment, the emitter and the detector are connected directly to the support base, which can preferably be a semiconductor layer. This way, each cell represents a resilient bell-structured shell that defines the housing and fits the support base.

Alternatively, each cell comprises a closed bottom to form a closed container in which the emitter and the detector are present. Even in this case, each cell is then arranged on the support base, in order to form the sensorized support surface.

Advantageously, said cells have a truncated pyramid-structure with a square or rectangular base. In particular, said cells fill the base, and have oblique side walls that end on said support wall.

The truncated pyramid structure is stiff with respect to tangential loads bearing on the side walls, and deformable with respect to normal loads bearing on the support wall. Such a structure allows then to minimize the expansion of the side walls, and enables the arrangement on the support base of a high number of sensors close to each other improving remarkably the resolution of the sensorized mat.

Furthermore, the truncated pyramid structure in combination with the optical sensors allows that the measurement signals have a range/signal excursion that is high enough to be directly transmitted and analysed by the control unit.

The predetermined separation space S between adjacent cells increases in height, between a minimum value and a maximum value, so that at the truncated pyramid base, the cells are substantially adjacent to each other and at the truncated pyramid top the cells are spaced. This way, since the expansion of the truncated pyramid cells is carried out in the intermediate zone of the frustum of pyramid, it occurs without that there is in any case substantial contact between the side walls of the frustum of pyramid.
- The separator enables the sensor to be extremely unaffected by tangential deformation aligned to the same element.

Furthermore, the combination between the optical sensor including the light emitter with the photosensitive detector and the pyramid structure of the cell with separation element, allow to obtain output measurement signals already comprising a range/signal excursion that is high enough to be directly transmitted and analysed. In particular, the output signal has a broad dynamic range (maximum signal excursion), therefore the electronic that can be used for treating the signal is simplified. This way, the electronics mounted to the mat is reduced to the minimum so that it can be completely integrated in the sensorized mat.

This combination of structure of cell and sensors generates very broad outputs, such that the sensitivity of the sensor to environmental conditions (temperature, humidity, *drift* time) is minimum. This way, the sensor does not require re-calibrations following to the first use.

In particular said separation is oriented in a direction which is the same as most of the tangential forces; in the case of the analysis of the gait, it is oriented along the direction of the same. This way, the sensor is substantially insensitive to tangential forces along the direction of the gait developed between the foot and the sole.

Alternatively, each cell comprises in said housing N emitters and M receivers equipped with K separation elements. In this case, it is possible to determine, in the most accurate way, the normal force also in the presence of tangential force, and for determining also the tangential force.

In a particular exemplary embodiment, said sensorized mat is a sensorized insole configured to fit a shoe for monitoring the pressures developed by the foot of a user.

Advantageously, said sensorized insole comprises a housing arranged at the plantar arch for said control unit and said supply unit. This way, the housing obtained at the plantar arch, has the advantage to exploit an area which is not subject to plantar pressures (in healthy subjects). In this area it is possible then to integrate all the electronics for data acquisition and signal treatment, in order to obtain an insole with electronics on board.

In particular, said control unit comprises a means for transmitting the measurement signals measured by said sensors towards a remote unit, for example a Bluetooth emitter. The latter is put in the housing within the plantar arch, in particular in position orthogonal with respect to the insole.

In particular, a coating layer is provided that comprises said plurality of cells arranged to couple with said support base where they are arranged said sensors. Preferably, said coating layer can be made of resilient material. In particular, the resilient material can be selected from the group consisting of:
- viscoelastic material, in particular silicone rubber;
- plastic material;
- synthetic rubber;
- natural rubber;
- or a combination thereof.

This way, the resilient material has the advantage of being soft and ergonomic, and of having a damping effect. Furthermore, the ergonomic advantage is not hindered by further external layers, which do not need to be added to the sensor, like the insoles of the prior art, but by the same cell structure of the sensors, which is ergonomic and soft as such, as well as by the box-like shape of each cell in combination with the resilient material used.

Advantageously, said coating layer of said cells is a dark colour shielding, in particular black, in order to avoid that the external light affects the device or that light leaks into the environment.

According to another aspect of the invention a method is provided for measuring a load acting on a surface, comprising the steps of:
- prearranging a support base;
- prearranging in a distributed way on said support base a plurality of sensors adapted to generate respective measurement signals when they are subjected to said load bearing on them, wherein each sensor comprises a deformable cell with box-like shape, each of said cells comprising:
   - a support wall that is opposite, in use, to said support base and arranged to receive said load, and a plurality of deformable side walls located between said base and said support wall that define a housing;
   - at least one light emitter and at least one photosensitive detector in said housing,
- and wherein said plurality of sensors is arranged on said support base so that said cells are adjacent to each other and separated from each other by a predetermined separation space,
- measuring by means of each photosensitive detector of each cell a change of light intensity collected from a respective emitter of said cell owing to the deformation of said cell when it is subjected to said load,
wherein said separation space between two adjacent cells is such that, when said support wall of each cell is subjected to said load, said side walls change their shape and are compressed towards said support base and, while expanding laterally occupy a deformation space less than said separation space, so that adjacent cells do not touch each other.
wherein a matrix of cells having truncated pyramid-structure is provided, in such a way that:
- said truncated pyramid structure is stiff with respect to tangential loads bearing on said side walls, and deformable with respect to normal loads bearing on said support wall;
- said truncated pyramid structure in combination with said sensors allows that said measurement signals have a broad dynamic range that is high enough to be directly transmitted and analysed by said control unit;
wherein each truncated pyramid cell has a stepped portion so that:
- stepped portions of adjacent truncated pyramid cells are substantially adjacent to each other;
- a minimum separation space Smin is defined at said stepped portion, and a maximum separation space Smax is defined at said support wall;
and wherein each of said cells comprises:
- said emitter of a light source;
- said photosensitive detector arranged opposite said emitter;
- a separation element located between said emitter and said photosensitive detector and arranged to move from a rest configuration, in which said cell is not subject to said load and all the light emitted by said emitter is collected by said photosensitive detector, to a bearing configuration, in which said separation element is lowered proportionally to said load and is located between said emitter and said photosensitive detector partially masking the light directed towards said photosensitive detector, proportionally to said load.

### Brief description of the drawings

The invention will be now shown with the following description of an exemplary embodiment thereof, exemplifying but not limitative, with reference to the attached drawings in which:
- Fig. 1 shows a perspective view of a sensorized mat according to the invention, having a matrix of sensorized cells;
- Fig. 2 shows a diagrammatical cross sectional view of a sensorized cell having a light emitter and a detector as well as a separation element which is adapted to shield the emitted light responsive to the load that acts on the cell;
- Fig. 3 shows a diagrammatical cross sectional view of a couple of cells adjacent to each other with frustum shape, separated by a separation space, which compares the behaviour of a cell when it is subjected to a load and highlights that the deformation of the side walls does not create effect of cross-talk with an adjacent cell;
- Fig. 4A diagrammatically shows a perspective view of a mat, or mattress, in which the structure of sensorized mat shown in Figs. 1 to 3 can be implemented;
- Fig. 4B diagrammatically shows a perspective view of a chair in which the sensorized mat, according to the invention, can be installed at the seat and/or the back rest;
- Fig. 4C shows a perspective diagrammatical view of a sensorized insole comprising the sensorized cells of Fig. 2, comprising a support base coupled to a cover of resilient material with a matrix of deformable cells arranged to define a support surface for the foot of a user;
- Fig. 5A diagrammatically shows the semiconductor support base comprising a plurality of zones and having a housing at the plantar arch for the electronics;
- Fig. 5B diagrammatically shows the semiconductor support base of Fig. 5, in which a plurality of emitters and receivers are arranged on each zone and where a electronic board is provided which controls and supplies the sensors;
- Fig. 6 shows a top plan view of a sensorized insole arranged in a shoe that depicts the plurality of sensorized cells adjacent to each other that cover the whole foot support area.

### Description of the forms of preferred embodiment.

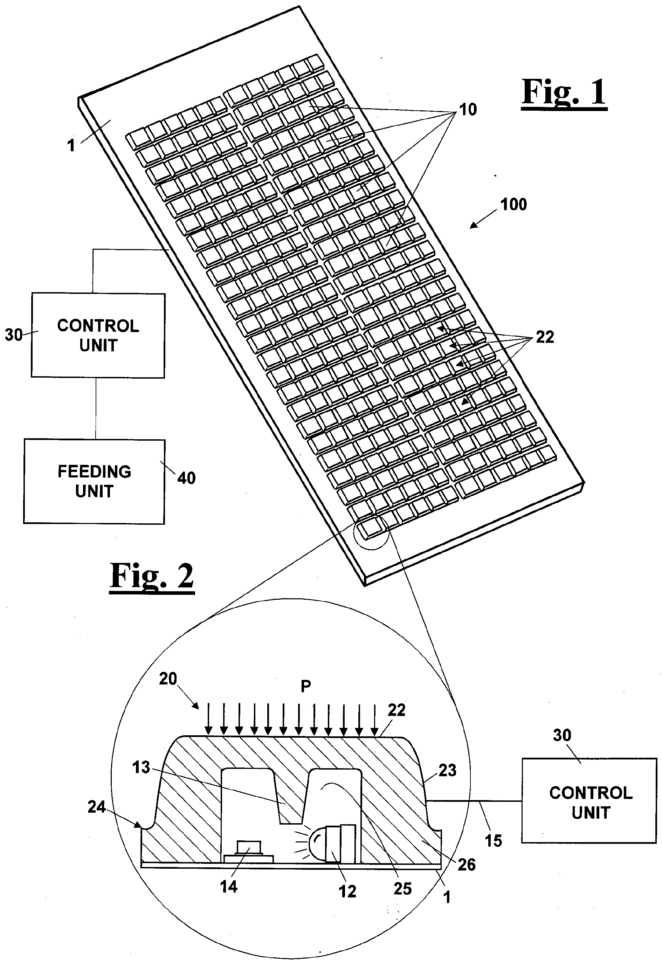

In Fig. 1 a structure of sensorized mat 100 is diagrammatically shown, comprising a support base 1 where a plurality of sensors 10 is arranged, which is adapted to generate corresponding measurement signals 15 when the sensors are subjected to a load P bearing on them (Fig.2).

The structure of mat 100 also comprises a control unit 30 associated with the plurality of sensors 10 and configured to analyse and/or transmit the measurement signals 15, as well as a supply unit 40 arranged to supply the plurality of sensors 10 and the control unit 30.

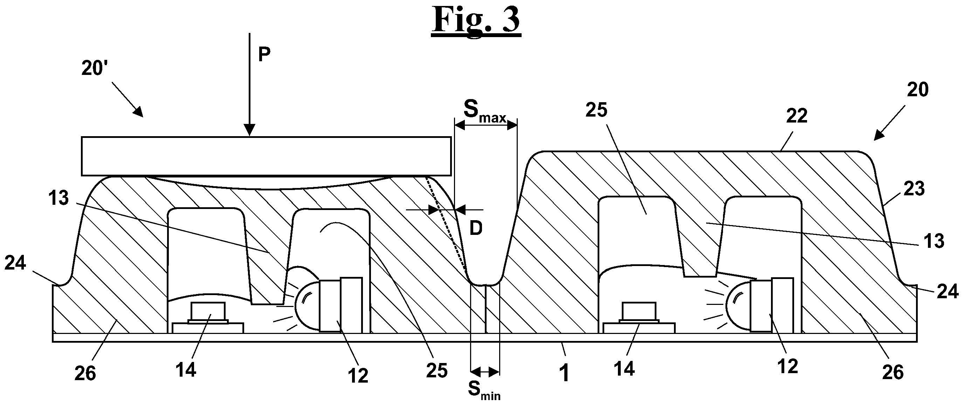

In particular, as shown in the enlarged view of Fig. 2, each sensor 10 comprises a deformable cell 20 with box-like shape, and the plurality of sensors 10 is arranged on a support base 1, in particular made of a resin of the type commonly used for boards electronic, so that the cells 20 are adjacent to each other and separated by a predetermined separation space S (Fig.3). The predetermined separation space (S) present between two cells 20 adjacent, in particular for small loads, avoids that the sensors arranged adjacently can touch each other touching each other. This way, it is avoided the risk of affecting the measurements.

More in particular, each cell 20 comprises a support wall 22 which is opposite, in use, to support base 1 and is arranged to receive a load P. Furthermore, the cell comprises a plurality of side walls 23 which, along with support wall 22 and support base 1, define, in use, a chamber, or space, or housing 25, in which at least one light emitter 12 and at least one photosensitive detector 14 are housed, the latter configured to measure a changing light intensity owing to the deformation of each cell 20 when it is subjected to the load P.

This way, as shown in Fig. 3, when the support wall 22 of each cell 20 is subjected to load P, the side walls 23 change their shape and are compressed towards support base 1 while expanding laterally, occupying a deformation space D less than the separation space S.

In particular, in Fig. 3, a comparison is shown between a cell 20' subject to the load P and a cell 20 which is not subject to the load. The shape of the cells 20 and 20' is such that the side walls 23 are expanded, shown with discontinuous line, with respect to a rest configuration that does not occupy the separation space S, thus avoiding to deform the adjacent cell, and then cause a "cross-talk" which affects the measurement. This way, it is ensured a most accurate measurement of the pressure generated by the foot when walking, or in a static standing position. In fact, only the cells 20' subject to a load P generate a corresponding measurement signal 15, whereas the adjacent cells are not affected, since the deformation D of the side walls 23 of each cell is less than the separation space S set between the cells 20, 20' same. In other words, the cells not directly subject to the load P are not involved for a same action of deforming the cells under the load, and not generate then a corresponding measurement signal. In addition, the cells with box-like shape are an array of resilient dampers allowing a damping effect, without requiring further damping layers above the insole.

In particular, each cell 20 comprises a boundary frame 26, of resilient material, which defines the housing 25 in which the emitter 12 and the photosensitive detector 14 are arranged. The boundary frame 26 is adapted to contain electronic components, for example acquisition/conditioning components.

More in particular, such a shape of the cells 20 causes, for the presence of the side walls 23 and of their thickness, the inner areas 25 within sensor 10 to be surrounded, in a top plan view, by the boundary frame 26 (Fig.2) not occupied by the emitter 12 and by the detector 14. In particular, each cell 20 comprises a "empty" part 25, which represents the housing and a "full" part filled 26 of resilient material, in particular silicone rubber, glued to support base 1. The solid parts 26 form, if seen in top plan view, a 'matrix of frames'. All this residual area can be used advantageously, as described above, for housing in a distributed way electronic components useful, for example, for treating or transmitting the measured data.

More in particular, as shown in Figs. 2 and 3, each deformable cell 20 has a truncated pyramid-structure, in particular a square based truncated pyramid-structure, or rectangular. Such a shape minimizes the expansion of the side walls 23, and enables the arrangement on support base 1 of a high number of sensors close to each other improving remarkably the resolution of the sensorized mat. Furthermore, the truncated pyramid structure as such is very stiff to tangential loads and not much stiff to normal loads. Therefore the sole structure determines substantial deformation to the normal loads of interest, and low deformation to the tangential loads.

The structure of mat 100 is "tiled" by means of cells 20 with truncated pyramid-structure, each of which has a stepped portion 24 that makes it possible to obtain a minimum separation space Sₘᵢₙ at the base of each chamber 25 defined by each cell 20 with support base 1, so that the deformation of the next cell 20 only occupies this space not involving the next cell. This way, the predetermined separation space S between adjacent cells increases versus height, between a value Sₘᵢₙ and a value Sₘₐₓ, so that at the stepped portion 24 of the frustum of pyramid, the cells are substantially adjacent to each other, and at the truncated pyramid top 22 the cells are sensibly distant from each other (Fig.3). This way, the expansion of the truncated pyramid cells is carried out in the intermediate zone of the frustum of pyramid, without that there is in any case substantial contact between the side walls 23 of the truncated pyramid cells.

In particular, each cell 20 comprises a single light emitter 12 and a respective photosensitive detector 14 arranged at opposite sides with respect to each other. Among them a separation element 13 is provided, obtained in frame 26, that is arranged to move from a rest configuration, where it allows detector 14 to capture all the light radiation emitted by source 12, to a bearing configuration (Fig.3), where separation element 13 lowers proportionally to the load P and is located between the source 12 and the detector 14, allowing to obtain a changing light intensity proportional to the load P. This way, the separator 13 enables the sensor to be extremely unaffected by tangential deformation aligned to the element same.

The combination between the optical sensor including the light emitter 12 with the photosensitive detector 14 and the pyramid structure of the cell 20 with separation element 13, allows to obtain output measurement signals already comprising a range/signal excursion that is high enough to be directly transmitted and analysed. In particular, the output signal has a broad dynamic range (maximum signal excursion), therefore the electronic that can be used for treating the signal is simplified. This way, the electronics mounted to the mat is reduced to the minimum so that it can be completely integrated in the sensorized mat.

Furthermore, the combination of the cell structure 20 and of the optical sensors generates very broad outputs, and minimizes the sensitivity of the sensor to environmental conditions (temperature, humidity, drift time). This way, the sensor does not require re-calibration following to the first use. This allows making acquisitions in ecological places (household use, or other) for long periods without the aid of operators and special machines.

In particular, the orientation of separation element 13 is coincident to the direction towards which most of the tangential forces are directed. In the case of analysis of the gait, it is oriented along the direction of the same. This way, the sensor is substantially insensitive to tangential forces with respect to the direction of the gait, which are developed between the foot and the sole.

Alternatively, each cell comprises, within a housing 25, N emitters 12 and M receivers 14 equipped with K separation elements 13. In this case, it is possible to determine, in the most accurate way, the normal force also in the presence of tangential forces, and for determining also the tangential forces.

The sensorized mat 100, as described above, and shown in Figs. 1 to 3, can be used in a variety of types of applications with the same purpose of measuring the pressures, in particular the distributed pressures on it applied.

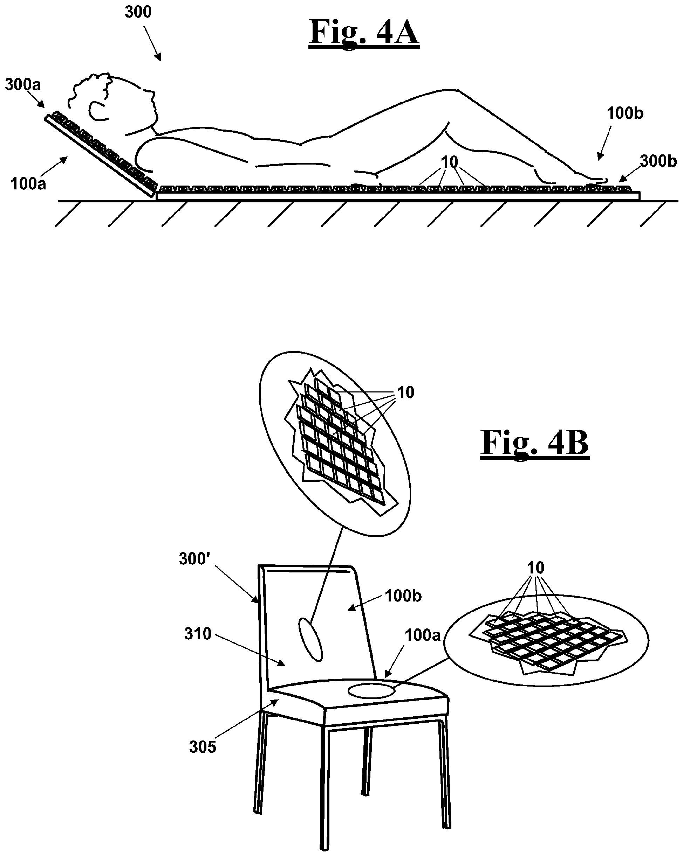

For example, the sensorized mat 100 can be integrated in a mat, or mattress 300, on which a user can lay (see Fig. 4A). In particular a first sensorized mat 100a at the back rest 300a and a second mat 100b at the couch 300b can be provided.

In another exemplary embodiment and diagrammatically shown in Fig. 4B, the sensorized mat 100 can be put at the back rest 300'a and/or at the seat 300'b of a chair 300', or an armchair. Normally, the mat 100 can be integrated, or implemented, in other devices or products, not shown in the figures, for which a pressure has to be measured, in particular a distributed pressure.

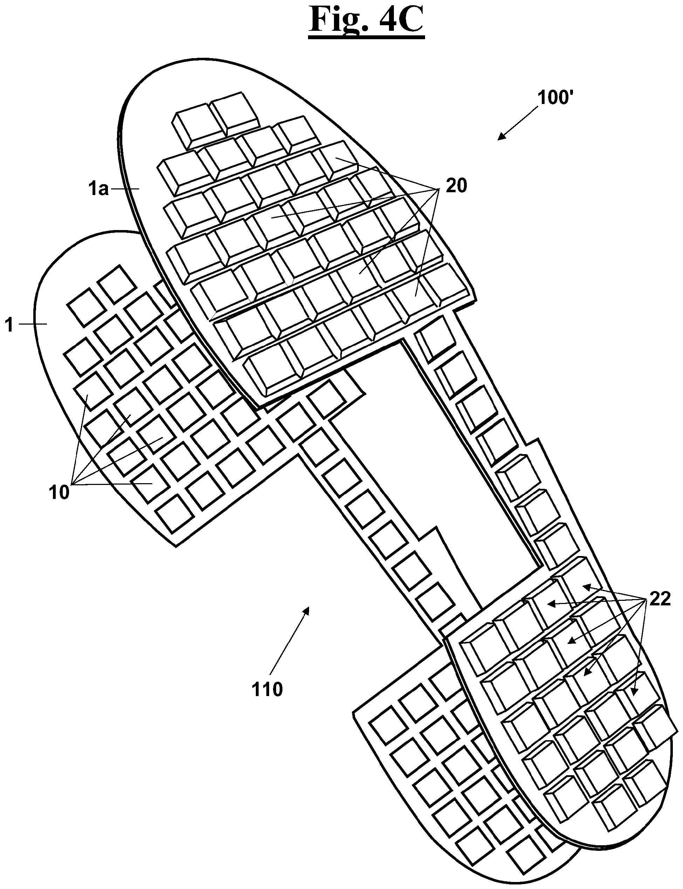

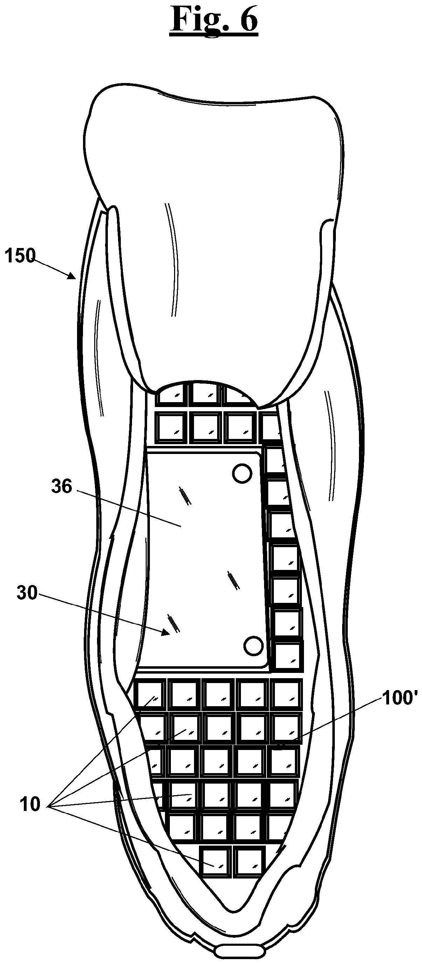

In a particular in the exemplary embodiment of Fig. 4C, the sensorized mat is a sensorized insole 100' configured to fit a shoe 150 (Fig.6) for monitoring the pressures developed by the foot of a user.

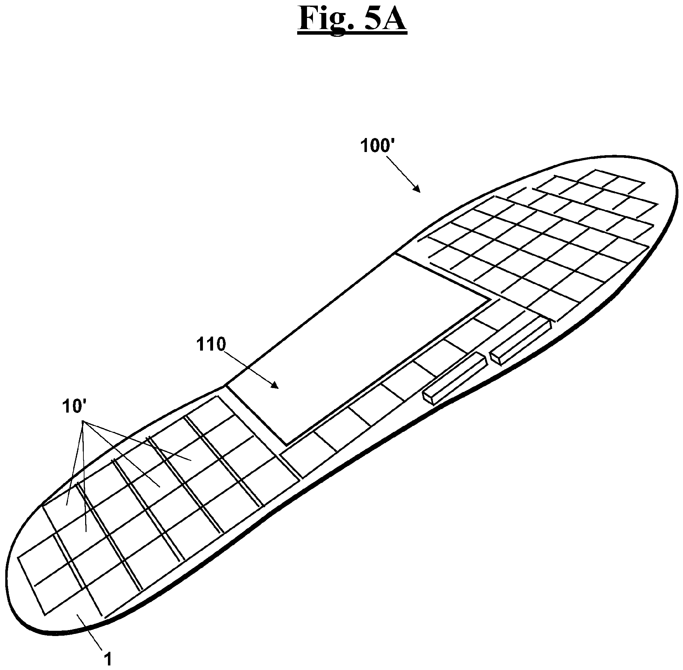

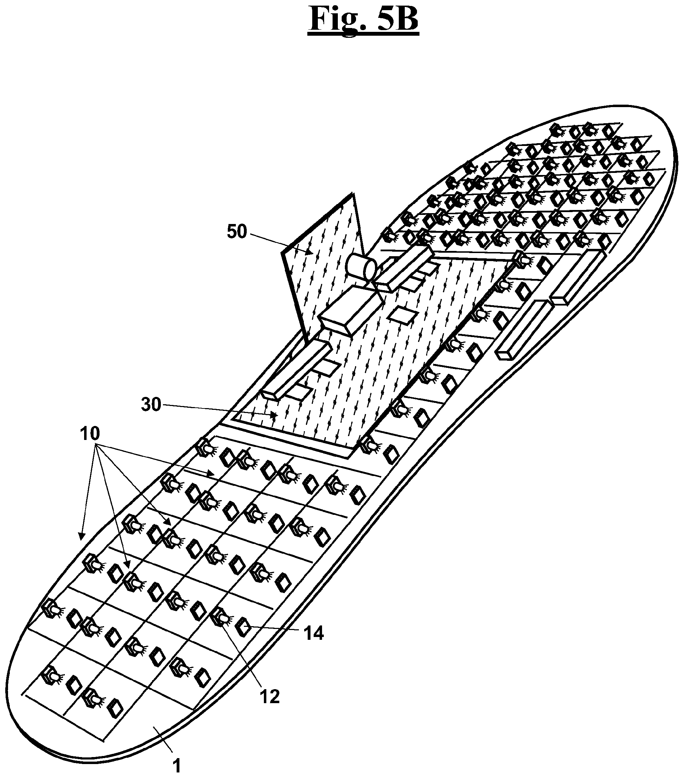

From a structural viewpoint, the sensorized insole 100' comprises the semiconductor support base 1, as shown in Fig. 5, for example made by moulding, which comprises a plurality of zones 10' where respective emitters 12 and receivers 14 (Fig. 5A) are arranged that are coupled to a coating layer 1a where a plurality of cells 20a are printed. Each cell 20 defines, then, a resilient bell-structured shell that is mounted to support base 1. In this case, then, as shown in Fig. 6, the emitter 12 and the detector 14 are connected directly to support base 1.

Alternatively, each cell 20 comprises a closed bottom and forms a closed container in which the emitter 12 and the detector 14 are present. Even in this case, each cell 20 is then arranged on support base 1, in order to form a tiled support surface.

Furthermore, support base 1 and coating layer 1a comprise a housing 110 at the plantar arch for controlling unit 30 and a supply unit 40 (Fig. 5) . The housing can be exploited for arranging transceiving means 50 that can transmit the data towards a remote unit, for example a Bluetooth emitter, associated with control unit 30. This way, the housing 110 obtained at the plantar arch has the advantage to exploit an area which is not subject to plantar pressures (in healthy subjects). In this area it is possible to integrate all the electronics for data acquisition and signal treatment, in order to obtain an insole with electronics on board. The transmission element 50 is advantageously located orthogonal with respect to the insole (Fig.5A), in order to exploit the free volume under the inner part of the plantar arch.

In particular, coating layer 1a is made of a resilient material. In particular, the resilient material can be selected from the group consisting of: viscoelastic materials, in particular silicone rubber, plastic materials, synthetic rubber, natural rubber or a combination thereof. This way, the resilient material has the advantage of being soft and ergonomic, and of having a damping effect. Furthermore, the ergonomic advantage is not hindered by further external layers, which do not need to be added to the sensor, like in the insoles of the prior art, and derives from the same cell structure of the sensors, which is ergonomic and soft as such, as well as from the box-like shape of each cell in combination with the resilient material used.

Coating layer 1a is a dark colour shielding in particular black, arranged to avoid that the external light affects photosensitive detector 14 or that the light emitted by the source 12 leaks into the environment.

Fig. 6 shows, furthermore, a stiff covering 36 located above housing 110 is arranged to cover the electronic controls 30 and the transceiving means 50.
From the foregoing description of various specific embodiments others, by applying current knowledge, will be able to modify and/or adapt in various applications such specific embodiments without further research and without parting from the scope of the invention as defined in the appended claims.

## Claims

1. A sensorized mat structure (100) comprising:
- a support base (1);
- a plurality of sensors (10) distributed on said support base (1) adapted to generate respective measurement signals (15) when they are subjected to a load (P) bearing on said sensorized mat structure (100);
- a control unit (30) associated with said plurality of sensors (10) configured to analyse and/or transmit said measurement signals (15) to a remote unit;
- a supply unit (40) arranged to supply said plurality of sensors (10) and said control unit (30); wherein each sensor (10) comprises a cell (20) with deformable box-like shape, and wherein said plurality of sensors (10) is arranged on said support base (1) so that said cells are adjacent with respect to each other,
each of said cells (20) comprising:
- a support wall (22), opposite, in use, to said support base (1), said support wall (22) arranged to receive said load (P),
- a plurality of deformable side walls (23), said plurality of side walls (23) and said support wall (22), in use, defining with said support base (1) a housing (25);
- at least one emitter (12) of a light source and at least one photosensitive detector (14) in said housing (25), said photosensitive detector (14) configured to measure a changing light intensity collected from said emitter (12) owing to the deformation of each cell (20) when it is subjected to said load (P);
wherein said cells (20) are separated from each other by a predetermined separation space (s) in such a way that, when said support wall (22) of each cell (20) is subjected to said load (P), said side walls (23) change their shape and are compressed towards said support base (1) and while expanding laterally occupy a deformation space (D) less than said separation space (S), so that adjacent cells do not touch each other;
wherein said cells (20) have a truncated pyramid-structure and define a matrix of cells, in such a way that:
- said truncated pyramid structure is stiff with respect to tangential loads bearing on said side walls (23), and deformable with respect to normal loads bearing on said support wall (22);
- said truncated pyramid structure in combination with said sensors (10) allows that said measurement signals (15) have a broad dynamic range that is high enough to be directly transmitted and analysed by said control unit (30);
wherein each truncated pyramid cell (20) having a stepped portion (24), wherein:
- stepped portions (24) of adjacent truncated pyramid cells (20) are substantially adjacent to each other;
- a minimum separation space (Smin) is defined at said stepped portion (24), and a maximum separation space (Smax) is defined at said support wall (22);
and wherein each of said cells (20) comprises:
- said emitter (12) of a light source;
- said photosensitive detector (14) arranged opposite said emitter (12);
- a separation element (13) located between said emitter (12) and said photosensitive detector (14) and arranged to move from a rest configuration (A), in which said cell (20) is not subject to said load (P) and all the light emitted by said emitter (12) is collected by said photosensitive detector (14), to a bearing configuration (B), in which said separation element (13) is lowered proportionally to said load (P) and is located between said emitter (12) and said photosensitive detector (14) partially masking the light directed towards said photosensitive detector (14), proportionally to said load (P).

2. A sensorized mat structure (100), according to claim 1, wherein each cell (20) comprises a boundary frame (26) of resilient material to define said housing (25) where said emitter (12) and said photosensitive detector (14) are located, said boundary frame (26) arranged to contain electronic components, for example components for acquisition and treatment of said measurement signals (15).

3. A sensorized mat structure (100), according to claim 1, wherein said cells (20) have a square-based or rectangular-based truncated pyramid-structure.

4. A sensorized mat structure (100), according to claim 1, adapted for analysis of the gait, said separation element (13) is oriented along the direction of the gait.

5. A sensorized mat structure (100), according to claim 1, wherein each cell (20) comprises in said housing (25) N emitters (12) and M receivers (14) equipped with K separation elements (13).

6. A sensorized mat structure (100), according to claim 1, wherein said sensorized mat (100) is a sensorized insole (100') configured to fit a shoe (150) for monitoring the pressures developed by the foot of a user.

7. A sensorized mat structure (100), according to claim 6, wherein said sensorized insole (100') comprises a housing (110) at the plantar arch for said control unit (30) and said supply unit (40).

8. A sensorized mat structure (100), according to claim 7, wherein said control unit (30) comprises transceiving means (50) for transmitting the measurement signals (15) measured by said sensors (10) towards a remote unit, in particular a Bluetooth emitter put in said housing (110) of the plantar arch.

9. A sensorized mat structure (100), according to claim 8, wherein said Bluetooth emitter is put in said housing (110) of the plantar arch in a position orthogonal with respect to said insole (100').

10. A sensorized mat structure (100), according to claim 1, wherein a coating layer (1a) is provided that comprises said plurality of cells (20) integrated to each other, said coating layer (1a) arranged to couple with said support base (1) where said sensors (10) are arranged, wherein preferably said coating layer (1a) is made of a resilient material.

11. A sensorized mat structure (100), according to claim 10, wherein said resilient material is selected from the group consisting of:
- viscoelastic material, in particular silicone rubber;
- plastic material;
- synthetic rubber;
- natural rubber; or a combination thereof.

12. A method for measuring a load (P) acting on a surface, comprising the steps of:
- prearranging a support base (1);
- prearranging in a distributed way on said support base (1) a plurality of sensors (10) adapted to generate respective measurement signals (15) when they are subjected to said load (P) bearing on said sensors (10), wherein each sensor (10) comprises a deformable cell (20) with box-like shape, each of said cells (20) comprising:
- a support wall (22) which is opposite, in use, to said support base (1) and arranged to receive said load (P);
- a plurality of deformable side walls (23) arranged, in use, between said support base (1) and said support wall (22) to define with said support base (1) a housing (25);
- at least one emitter (12) of a light source and at least one photosensitive detector (14) in said housing (25),
- and wherein said plurality of sensors (10) is arranged on said support base (1) so that said cells (20) are adjacent to each other and separated from each other by a predetermined separation space (S),
- measuring by means of each photosensitive detector (14) of each cell (20) a change of light intensity collected from a respective emitter (12) of said cell (20) owing to the deformation of said cell (20) when it is subjected to said load (P),
- wherein said separation space (S) between two adjacent cells (20) is such that, when said support wall (22) of each cell (20) is subjected to said load (P), said side walls (23) change their shape and are compressed towards said support base (1) and while expanding laterally occupy a deformation space (D) less than said separation space (S), so that adjacent cells do not touch each other;
wherein a matrix of cells (20) having truncated pyramid-structure is provided, in such a way that:
- said truncated pyramid structure is stiff with respect to tangential loads bearing on said side walls (23), and deformable with respect to normal loads bearing on said support wall (22);
- said truncated pyramid structure in combination with said sensors (10) allows that said measurement signals (15) have a broad dynamic range that is high enough to be directly transmitted and analysed by said control unit (30);
wherein each truncated pyramid cell (20) has a stepped portion (24), wherein:
- stepped portions (24) of adjacent truncated pyramid cells (20) are substantially adjacent to each other;
- a minimum separation space (Smin) is defined at said stepped portion (24), and a maximum separation space (Smax) is defined at said support wall (22);
and wherein each of said cells (20) comprises:
- said emitter (12) of a light source;
- said photosensitive detector (14) arranged opposite said emitter (12);
- a separation element (13) located between said emitter (12) and said photosensitive detector (14) and arranged to move from a rest configuration (A), in which said cell (20) is not subject to said load (P) and all the light emitted by said emitter (12) is collected by said photosensitive detector (14), to a bearing configuration (B), in which said separation element (13) is lowered proportionally to said load (P) and is located between said emitter (12) and said photosensitive detector (14) partially masking the light directed towards said photosensitive detector (14), proportionally to said load (P).

## Patentansprüche

1. Sensormattenstruktur (100), die Folgendes umfasst:
- einen Grundträger (1);
- eine Mehrzahl von auf dem Grundträger (1) verteilten Sensoren (10), die dafür konzipiert sind, Messsignale (15) zu erzeugen, wenn sie einer Last (P) ausgesetzt ist, die auf die Sensormattenstruktur (100) einwirkt;
- eine mit der Mehrzahl von Sensoren (10) verbundene Steuereinheit (30), die konfiguriert ist, um die Messsignale (15) zu analysieren und/oder an eine dezentrale Einheit zu senden;
- eine Versorgungsstromeinheit (40), die angeordnet ist, um die Mehrzahl von Sensoren (10) und die Steuereinheit (30) zu speisen; wobei jeder Sensor (10) eine Zelle (20) mit einer verformbaren Kastenform umfasst und wobei die Mehrzahl von Sensoren (10) so auf dem Grundträger (1) angeordnet ist, dass die Zellen zueinander benachbart sind,
wobei jede der Zellen (20) Folgendes umfasst:
- eine Stützwand (22), die sich im Gebrauch gegenüber von dem Grundträger (1) befindet, wobei die Stützwand (22) zur Aufnahme der Last (P) angeordnet ist,
- eine Mehrzahl von verformbaren Seitenwänden (23), wobei die Mehrzahl von Seitenwänden (23) und die Stützwand (22) im Gebrauch zusammen mit dem Grundträger (1) ein Gehäuse (25) bilden;
- wenigstens einen Emitter (12) einer Lichtquelle und wenigstens einen lichtempfindlichen Detektor (14) in dem Gehäuse (25), wobei der lichtempfindliche Detektor (14) konfiguriert ist, um eine von dem Emitter (12) erfasste veränderte Lichtintensität zu messen, die durch die Verformung jeder Zelle (20), wenn diese der Last (P) ausgesetzt ist, entsteht;
wobei die Zellen (20) durch einen vorher festgelegten Trennraum (s) derart voneinander getrennt sind, dass
wenn die Stützwand (22) von jeder Zelle (20) der Last (P) ausgesetzt ist, die Seitenwände (23) ihre Form ändern und in Richtung des Grundträgers (1) komprimiert werden und beim seitlichen Ausdehnen einen Verformungsraum (D) einnehmen, der kleiner ist als der Trennraum (S), sodass benachbarte Zellen einander nicht berühren;
wobei die Zellen (20) eine abgeschnitten-pyramidenförmige Struktur haben und eine Matrix von Zellen derart bestimmen, dass:
- die abgeschnitten-pyramidenförmige Struktur in Bezug auf tangentiale Lasten, die auf die Seitenwände (23) einwirken, starr und in Bezug auf normale Lasten, die auf die Stützwand (22) einwirken, verformbar ist;
- die abgeschnitten-pyramidenförmige Struktur in Verbindung mit den Sensoren (10) einen breiten Dynamikbereich der Messsignale (15) ermöglicht, der hoch genug ist, um von der Steuereinheit (30) direkt gesendet und analysiert zu werden;
wobei jede abgeschnitten-pyramidenförmige Zelle (20) einen gestuften Abschnitt (24) hat, wobei:
- die gestuften Abschnitte (24) von benachbarten abgeschnitten-pyramidenförmigen Zellen (20) im Wesentlichen zueinander benachbart sind;
- ein Mindesttrennraum (Smin) an dem gestuften Abschnitt (24) bestimmt ist und ein Höchsttrennraum (Smax) an der Stützwand (22) bestimmt ist und wobei jede der Zellen (20) Folgendes umfasst:
- den Emitter (12) einer Lichtquelle;
- den lichtempfindlichen Detektor (14), der gegenüber dem Emitter (12) angeordnet ist;
- ein Trennelement (13), das sich zwischen dem Emitter (12) und dem lichtempfindlichen Detektor (14) befindet und angeordnet ist, um sich aus einer Ruhekonfiguration (A), in der die Zelle (20) nicht der Last (P) ausgesetzt ist und das gesamte von dem Emitter (12) emittierte Licht von dem lichtempfindlichen Detektor (14) erfasst wird, in eine Einwirkungskonfiguration (B) zu bewegen, in der das Trennelement (13) proportional zu der Last (P) abgesenkt wird und sich zwischen dem Emitter (12) und dem lichtempfindlichen Detektor (14) befindet und dabei das in Richtung des lichtempfindlichen Detektors (14) gerichtete Licht proportional zu der Last (P) teilweise verdeckt.

2. Sensormattenstruktur (100) nach Anspruch 1, wobei jede Zelle (20) einen Begrenzungsrahmen (26) aus elastischem Material zur Bestimmung des Gehäuses (25) umfasst, in dem sich der Emitter (12) und der lichtempfindliche Detektor (14) befinden, wobei der Begrenzungsrahmen (26) angeordnet ist, um elektronische Komponenten zu enthalten, zum Beispiel Komponenten für die Erfassung und Verarbeitung der Messsignale (15).

3. Sensormattenstruktur (100) nach Anspruch 1, wobei die Zellen (20) eine abgeschnitten-pyramidenförmige Struktur mit quadratischer oder rechteckiger Grundfläche haben.

4. Sensormattenstruktur (100) nach Anspruch 1, dafür konzipiert, die Gangart zu analysieren, wobei das Trennelement (13) entlang der Gehrichtung ausgerichtet ist.

5. Sensormattenstruktur (100) nach Anspruch 1, wobei jede Zelle (20) in dem Gehäuse (25) N Emitter (12) und M Empfänger (14), versehen mit K Trennelementen (13), umfasst.

6. Sensormattenstruktur (100) nach Anspruch 1, wobei die Sensormatte (100) eine Sensoreinlegesohle (100') ist, die so angeordnet ist, dass sie in einen Schuh (150) passt, um die von dem Fuß eines Benutzers entwickelten Drücke zu überwachen.

7. Sensormattenstruktur (100) nach Anspruch 6, wobei die Sensoreinlegesohle (100') ein Gehäuse (110) am Fußsohlenbogen für die Steuereinheit (30) und die Versorgungsstromeinheit (40) umfasst.

8. Sensormattenstruktur (100) nach Anspruch 7, wobei die Steuereinheit (30) Sender-Empfänger-Mittel (50) für das Senden der von den Sensoren (10) gemessenen Messsignale (15) an eine dezentrale Einheit, insbesondere an einen in dem Gehäuse (110) des Fußsohlenbogens angeordneten Bluetooth-Emitter umfasst.

9. Sensormattenstruktur (100) nach Anspruch 8, wobei der Bluetooth-Emitter in einer gegenüber der Einlegesohle (100') orthogonalen Position in dem Gehäuse (110) des Fußsohlenbogens angeordnet ist.

10. Sensormattenstruktur (100) nach Anspruch 1, wobei eine Überzugsschicht (1a) vorgesehen ist, die die Mehrzahl von Zellen (20) ineinander integriert umfasst, wobei die Überzugsschicht (1a) zur Kopplung mit dem Grundträger (1) angeordnet ist, in dem die Sensoren (10) angeordnet sind, wobei die Überzugsschicht (1a) vorzugsweise aus einem elastischen Material besteht.

11. Sensormattenstruktur (100) nach Anspruch 10, wobei das elastische Material ausgewählt ist aus der Gruppe bestehend aus:
- viskoelastischem Material, insbesondere Silikonkautschuk;
- Kunststoff;
- synthetischem Kautschuk;
- Naturkautschuk oder einer Kombination daraus.

12. Verfahren für das Messen einer Last (P), die auf eine Oberfläche einwirkt, wobei das Verfahren die folgenden Schritte umfasst:
- Voranordnen eines Grundträgers (1);
- Voranordnen einer Mehrzahl von Sensoren (10) auf eine verteilte Weise auf dem Grundträger (1), wobei die Sensoren dafür konzipiert sind, um Messsignale (15) zu erzeugen, wenn sie der Last (P) ausgesetzt sind, die auf die Sensoren (10) einwirkt, wobei jeder Sensor (10) eine verformbare Zelle (20) mit einer Kastenform umfasst und wobei jede der Zellen (20) Folgendes umfasst:
- eine Stützwand (22), die sich im Gebrauch gegenüber von dem Grundträger (1) befindet und zur Aufnahme der Last (P) angeordnet ist;
- eine Mehrzahl von verformbaren Seitenwänden (23), die im Gebrauch zwischen dem Grundträger (1) und der Stützwand (22) angeordnet sind, um zusammen mit dem Grundträger (1) ein Gehäuse (25) zu bilden;
- wenigstens einen Emitter (12) einer Lichtquelle und wenigstens einen lichtempfindlichen Detektor (14) in dem Gehäuse (25),
- und wobei die Mehrzahl von Sensoren (10) derart auf dem Grundträger (1) angeordnet ist, dass die Zellen (20) zueinander benachbart und durch einen vorher festgelegten Trennraum (S) voneinander getrennt sind,
- Messen über jeden lichtempfindlichen Detektor (14) von jeder Zelle (20) einer von einem Emitter (12) der Zelle (20) erfassten Veränderung der Lichtintensität, die durch die Verformung der Zelle (20), wenn diese der Last (P) ausgesetzt ist, entsteht,
- wobei der Trennraum (S) zwischen zwei benachbarten Zellen (20) derart ist, dass, wenn die Stützwand (22) von jeder Zelle (20) der Last (P) ausgesetzt ist, die Seitenwände (23) ihre Form ändern und in Richtung des Grundträgers (1) komprimiert werden und beim seitlichen Ausdehnen einen Verformungsraum (D) einnehmen, der kleiner ist als der Trennraum (S), sodass benachbarte Zellen einander nicht berühren;
wobei eine Matrix von Zellen (20) mit einer abgeschnitten-pyramidenförmigen Struktur derart vorgesehen ist, dass:
- die abgeschnitten-pyramidenförmige Struktur in Bezug auf tangentiale Lasten, die auf die Seitenwände (23) einwirken, starr und in Bezug auf normale Lasten, die auf die Stützwand (22) einwirken, verformbar ist;
- die abgeschnitten-pyramidenförmige Struktur in Verbindung mit den Sensoren (10) einen breiten Dynamikbereich der Messsignale (15) ermöglicht, der hoch genug ist, um von der Steuereinheit (30) direkt gesendet und analysiert zu werden;
wobei jede abgeschnitten-pyramidenförmige Zelle (20) einen gestuften Abschnitt (24) hat, wobei:
- die gestuften Abschnitte (24) von benachbarten abgeschnitten-pyramidenförmigen Zellen (20) im Wesentlichen zueinander benachbart sind;
- ein Mindesttrennraum (Smin) an dem gestuften Abschnitt (24) bestimmt ist und ein Höchsttrennraum (Smax) an der Stützwand (22) bestimmt ist
und wobei jede der Zellen (20) Folgendes umfasst:
- den Emitter (12) einer Lichtquelle;
- den lichtempfindlichen Detektor (14), der gegenüber dem Emitter (12) angeordnet ist;
- ein Trennelement (13), das sich zwischen dem Emitter (12) und dem lichtempfindlichen Detektor (14) befindet und angeordnet ist, um sich aus einer Ruhekonfiguration (A), in der die Zelle (20) nicht der Last (P) ausgesetzt ist und das gesamte von dem Emitter (12) emittierte Licht von dem lichtempfindlichen Detektor (14) erfasst wird, in eine Einwirkungskonfiguration (B) zu bewegen, in der das Trennelement (13) proportional zu der Last (P) abgesenkt wird und sich zwischen dem Emitter (12) und dem lichtempfindlichen Detektor (14) befindet und dabei das in Richtung des lichtempfindlichen Detektors (14) gerichtete Licht proportional zu der Last (P) teilweise verdeckt.

## Revendications

1. Structure de matelas à capteur (100) comprenant :
- une base de support (1) ;
- une pluralité de capteurs (10) distribués sur ladite base de support (1) adaptée pour générer des signaux de mesure respectifs(15) lorsqu'ils sont soumis à une charge (P) appliquée sur ladite structure de matelas à capteur (100) ;
- une unité de commande (30) associée avec ladite pluralité de capteurs (10) configurée pour analyser et/ou transmettre lesdits signaux de mesure (15) à une unité éloignée ;
- une unité d'alimentation (40) agencée pour alimenter ladite pluralité de capteurs (10) et ladite unité de commande (30), chaque capteur (10) comprenant une cellule (20) à forme de boîte déformable, et ladite pluralité de capteurs (10) étant agencée sur ladite base de support (1) de sorte que les cellules soient adjacentes entre elles,
chacune desdites cellules (20) comprenant :
- une paroi de support (22) faisant face, en cours d'usage, à ladite base de support (1), ladite paroi de support (22) étant disposée pour recevoir ladite charge (P),
- une pluralité de parois latérales déformables (23), ladite pluralité de parois latérales (23) et ladite paroi de support (22) définissant, en cours d'usage, avec ladite base de support (1), un boîtier (25) ;
- au moins un émetteur (12) d'une source de lumière, et au moins un détecteur photosensible (14) dans ledit boîtier (25), ledit détecteur photosensible (14) étant configuré pour mesurer une intensité lumineuse changeante captée audit émetteur (12) en raison de la déformation de chaque cellule (20) lorsqu'elle est soumise à ladite charge (P) ;
lesdites cellules (20) étant séparées l'une de l'autre par un espace de déformation prédéterminé (S) de sorte que lorsque chaque paroi de support (22) de chaque cellule (20) est soumise à ladite charge (P), lesdites parois latérales (23) changent leur forme et sont comprimées en direction de ladite base de support (1), et occupent, tout en se dilatant latéralement, un espace de déformation (D) inférieur audit espace de séparation (S), de sorte que des cellules adjacentes ne se touchent pas ;
lesdites cellules (20) présentant une structure à pyramide tronquée, et définissant une matrice de cellules, de sorte que :
- ladite structure à pyramide tronquée soit rigide relativement à des charges tangentielles appliquées sur lesdites parois latérales (23), et déformable relativement à des charges normales appliquées sur ladite paroi de support (22) ;
- ladite structure à pyramide tronquée, combinée avec lesdits capteurs (10), donne auxdits signaux de mesure (15) une large plage dynamique suffisamment haute pour être transmise et analysée directement par ladite unité de commande (30) ;
chaque cellule à pyramide tronquée (20) présentant une partie étagée (24), dans laquelle :
- des parties étagées (24) de cellules à pyramide tronquée (20) sont substantiellement adjacentes l'une à l'autre ;
- un espace de déformation minimum (Smin) est défini à ladite partie étagée (24), et un espace de déformation maximum (Smax) est défini à ladite paroi de support (22) ;
et chacune desdites cellules (20) comprenant :
- ledit émetteur (12) d'une source de lumière ;
- ledit détecteur photosensible (14) agencé en face dudit émetteur (12) ;
- un élément de séparation (13) placé entre ledit émetteur (12) et ledit détecteur photosensible (14), et agencé pour se déplacer d'une configuration de repos (A), dans laquelle ladite cellule (20) n'est pas soumise à ladite charge (P) et l'intégralité de la lumière émise par ledit émetteur (12) est prélevée par ledit détecteur photosensible (14), à une configuration porteuse (B), dans laquelle ledit élément de séparation (13) est baissé proportionnellement à ladite charge (P), et est situé entre ledit émetteur (12) et ledit détecteur photosensible (14), en masquant partiellement la lumière dirigée vers ledit détecteur photosensible (14), proportionnellement à ladite charge (P).

2. Structure de matelas à capteur (100) selon la revendication 1, chaque cellule (20) comprenant un cadre limite (26) d'un matériau élastique pour définir ledit boîtier (25) où se trouvent ledit émetteur (12) et ledit détecteur photosensible (14), ledit cadre limite (26) étant agencé pour contenir des composants électroniques, par exemple des composants pour l'acquisition et le traitement desdits signaux de mesure (15).

3. Structure de matelas à capteur (100) selon la revendication 1, lesdites cellules (20) présentant une structure à pyramide tronquée à base carrée ou à base rectangulaire.

4. Structure de matelas à capteur (100) selon la revendication 1, adaptée pour la mesure de la démarche, ledit élément de séparation (13) étant orienté le long de la direction de la démarche.

5. Structure de matelas à capteur (100) selon la revendication 1, chaque cellule (20) comprenant, dans ledit boîtier (25), des émetteurs N (12) et des récepteurs M (14) équipés d'éléments de séparation K (13).

6. Structure de matelas à capteur (100) selon la revendication 1, ledit matelas à capteur (100) étant un semelle à capteur (100') configurée pour tenir dans une chaussure (150) pour contrôler les pressions produites par le pied d'un utilisateur.

7. Structure de matelas à capteur (100) selon la revendication 6, ladite semelle à capteur (100') comprenant un boîtier (110) sur la voûte plantaire pour ladite unité de commande (30) et ladite unité d'alimentation (40).

8. Structure de matelas à capteur (100) selon la revendication 7, ladite unité de commande (30) comprenant un dispositif d'émission réception (50) pour la transmission, à une unité éloignée, en particulier un émetteur Bluetooth placé dans ledit boîtier (110) de la voûte plantaire, des signaux de mesure (15) mesurés par lesdits capteurs (10).

9. Structure de matelas à capteur (100) selon la revendication 8, ledit émetteur Bluetooth état placé dans ledit boîtier (110) de la voûte plantaire dans une position orthogonale relativement à ladite semelle (100').

10. Structure de matelas à capteur (100) selon la revendication 1, comprenant une couche de revêtement (1a) composée de ladite pluralité de cellules (20) intégrée l'une à l'autre, ladite couche de revêtement (1a) étant agencée pour s'accoupler avec ladite base de support (1), où lesdits capteurs (10) sont agencés, ladite couche de revêtement (1a) étant de préférence réalisée avec une matière élastique.

11. Structure de matelas à capteur (100) selon la revendication 10, ladite matière élastique étant sélectionnée dans un groupe composé :
- d'une matière viscoélastique, en particulier du caoutchouc silicone ;
- d'une matière plastique ;
- de caoutchouc synthétique ;
- de caoutchouc naturel ; ou d'une combinaison de ces derniers.

12. Méthode de mesure d'une charge (P) agissant sur une surface, comprenant les étapes suivantes :
- pré-agencement d'une base de support (1) ;
- pré-agencement, de façon distribuée sur ladite base de support (1), d'une pluralité de capteurs (10) adaptée pour générer des signaux de mesure respectifs(15) lorsqu'ils sont soumis à ladite charge (P) appliquée sur lesdits capteurs (10), chaque capteur (10) comprenant une cellule (20) déformable en forme de boîte , chacun desdites cellules (20) comprenant :
- une paroi de support (22) faisant face, en cours d'usage, à ladite base de support (1), et agencée pour recevoir ladite charge (P) ;
- une pluralité de parois latérales déformables (23) agencées, en cours d'usage, entre ladite base de support (1) et ladite paroi de support (22) pour définir, avec ladite base de support (1), un boîtier (25) ;
- au moins un émetteur (12) d'une source de lumière, et au moins un détecteur photosensible (14) dans ledit boîtier (25),
- et ladite pluralité de capteurs (10) étant agencée sur ladite base de support (1), de sorte que lesdites cellules (20) soient adjacentes entre elles et séparées l'une de l'autre par un espace de séparation prédéterminé (S),
- mesurant, à l'aide de chaque détecteur photosensible (14) de chaque cellule (20) un changement d'intensité lumineuse captée d'un émetteur correspondant (12) de ladite cellule (20) dû à la déformation de ladite cellule (20) lorsqu'elle est soumise à ladite charge (P),
- ledit espace de séparation (S) entre deux cellules adjacentes (20) étant tel que lorsque chaque paroi de support (22) de chaque cellule (20) est soumise à ladite charge (P), lesdites parois latérales (23) changent leur forme et sont comprimées en direction de ladite base de support (1), et occupent, tout en se dilatant latéralement, un espace de déformation (D) inférieur audit espace de séparation (S), de sorte que des cellules adjacentes ne se touchent pas ;
- une matrice de cellules (20) présentant une structure à pyramide tronquée est fournie, de telle façon que :
- ladite structure à pyramide tronquée soit rigide relativement à des charges tangentielles appliquées sur lesdites parois latérales (23), et déformable relativement à des charges normales appliquées sur ladite paroi de support (22) ;
- ladite structure à pyramide tronquée, combinée avec lesdits capteurs (10), donne auxdits signaux de mesure (15) une large plage dynamique suffisamment haute pour être transmise et analysée directement par ladite unité de commande (30) ;
chaque cellule à pyramide tronquée (20) présentant une partie étagée (24), dans laquelle :
- des parties étagées (24) de cellules à pyramide tronquée (20) adjacentes sont substantiellement adjacentes l'une à l'autre ;
- un espace de déformation minimum (Smin) est défini à ladite partie étagée (24), et un espace de déformation maximum (Smax) est défini à ladite paroi de support (22) ;
et chacune desdites cellules (20) comprenant :
- ledit émetteur (12) d'une source de lumière ;
- ledit détecteur photosensible (14) agencé en face dudit émetteur (12) ;
- un élément de séparation (13) placé entre ledit émetteur (12) et ledit détecteur photosensible (14), et agencé pour se déplacer d'une configuration de repos (A), dans laquelle chaque cellule (20) n'est pas soumise à ladite charge (P) et l'intégralité de la lumière émise par ledit émetteur (12) est prélevée par ledit détecteur photosensible (14), à une configuration porteuse (B), dans laquelle ledit élément de séparation (13) est baissé proportionnellement à ladite charge (P), et est situé entre ledit émetteur (12) et ledit détecteur photosensible (14), en masquant partiellement la lumière dirigée vers ledit détecteur photosensible (14), proportionnellement à ladite charge (P).
